Europäisches Patentamt

European Patent Office

Office européen des brevets

(19)

(11) Veröffentlichungsnummer: **0 344 554**

**A2**

(12) # EUROPÄISCHE PATENTANMELDUNG

(21) Anmeldenummer: 89109120.9

(51) Int. Cl.4: **G01N 5/02 , G01N 33/34**

(22) Anmeldetag: 20.05.89

(30) Priorität: 28.05.88 DE 3818199

(43) Veröffentlichungstag der Anmeldung:
06.12.89 Patentblatt 89/49

(84) Benannte Vertragsstaaten:
DE FR SE

(71) Anmelder: **BASF Aktiengesellschaft
Carl-Bosch-Strasse 38
D-6700 Ludwigshafen(DE)**

(72) Erfinder: **Benninghoff, Norbert
Holzweg 73A
D-6719 Mertesheim(DE)**
Erfinder: **Jaensch, Manfred
Gluckstrasse 15
D-6720 Speyer(DE)**
Erfinder: **Melzer, Jaroslav
Kirchenstrasse 116
D-6700 Ludwigshafen(DE)**
Erfinder: **Niesar, Gunter
Buschstrasse 44
D-6708 Neuhofen(DE)**
Erfinder: **Schuster, Werner
Heinrich-Heine-Strasse 6
D-6733 Hassloch(DE)**

(54) **Messanordnung zur Bestimmung der Flüssigkeitsaufnahme von Papier.**

(57) Die Meßanordnung besteht aus aufeinanderfolgenden Arbeitsstationen (I-V), die einen Vorrat (2) an Papierproben (3), eine Waage (9), eine mechanisierte Befeuchtungseinrichtung (10-19) für die Probe und eine ebenfalls mechanisierte Ablöscheinrichtung (22) für die befeuchtete Probe umfassen. Für die Durchführung der Messung ist ein von einem Arbeitsprogramm steuerbarer, mit einer Greifvorrichtung (4, 7) ausgestatteter Industrieroboter (1) vorgesehen.

EP 0 344 554 A2

## Meßanordnung zur Bestimmung der Flüssigkeitsaufnahme von Papier

Die Erfindung betrifft eine Meßanordnung zur Bestimmung der Flüssigkeitsaufnahme von Papier, bestehend aus aufeinanderfolgenden Arbeitsstationen, die eine Waage für eine Papierprobe, eine Befeuchtungseinrichtung für die Papierprobe, die durch einen Metallzylinder und eine mit der Papierprobe diesen abschließbaren Platte gebildet ist, und eine Ablöscheinrichtung mit einem oberen und unteren, die Papierprobe einschließenden Löschkarton und eine Ablöschrolle aufweisen.

Zur Beurteilung der Flüssigkeitsaufnahme von Papier oder Pappe sind das Meßverfahren und die Geräte dafür nach Cobb nach DIN 53 132 bzw. ISO 535-1976 bekannt. Mit der danach aufgebauten, oben beschriebenen Meßanordnung ist das Meßverfahren nur von Hand durchführbar. Neben einem hohen Arbeits- und Zeitaufwand ist dabei von Nachteil, daß das Meßergebnis sehr von der Genauigkeit bei den einzelnen Arbeitsschritten und damit von der jeweiligen Bedienungsperson abhängt.

Es bestand daher die Aufgabe, eine Meßanordnung für ein Meßverfahren nach der oben genannten Norm zu entwickeln, mit der das Meßverfahren vollmechanisiert durchführbar ist.

Die die Aufgabe lösende Meßanordnung entsprechend dem eingangs beschriebenen Aufbau weist folgende Merkmale auf:
- eine von einem Arbeitsprogramm steuerbare, in den Raumkoordinaten den einzelnen Arbeitsstationen zufahrbare Einrichtung mit einer Greifvorrichtung,
- eine der Waage vorgeordnete Vorratseinrichtung für die Papierproben,
- der Metallzylinder und die Platte sind mittels eines Arbeitszylinders aufeinander zufahrbar, wobei der Metallzylinder schwenkbar gelagert ist,
- der obere und untere Löschkarton ist jeweils in Form einer mit einem Materialvorschub verbundenen Bahn über eine Auflagefläche geführt, wobei auf der oberen Bahn die Ablöschrolle mittels eines Reversierantriebs abrollbar ist.

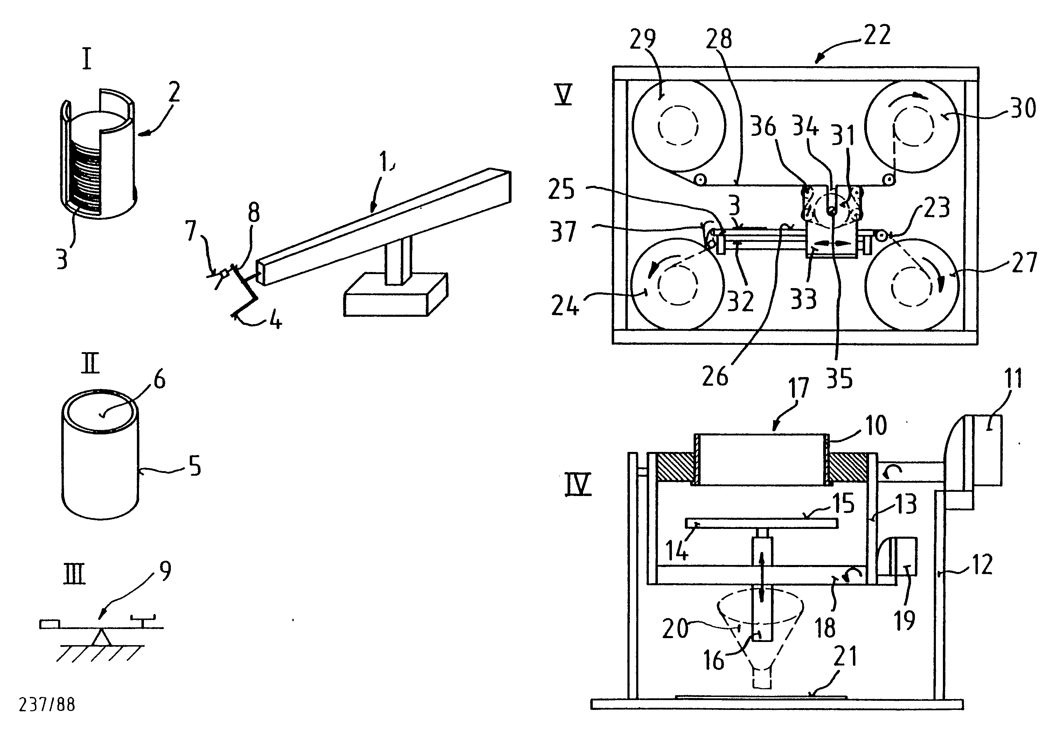

Nachfolgend ist die erfindungsgemäße Meßanordnung anhand eines in der Zeichnung schematisch dargestellten Ausführungsbeispiels näher beschrieben.

Zentrales Teil der Meßanordnung ist eine von einem Arbeitsprogramm steuerbare und in den Raumkoordinaten (X, Y, Z) den einzelnen, darum gruppierten Arbeitsstationen I-V zufahrbare Einrichtung 1. Hierfür wird ein im Fachhandel zur Verfügung stehender Industrieroboter mit der zugehörigen Steuerung eingesetzt, deren näheren Erläuterungen den entsprechenden Geräteunterlagen entnommen werden können.

Die Arbeitsstation I wird durch eine Vorratseinrichtung 2, beispielsweise einen seitlich geöffneten Zylinder, für kreisförmige Papierproben 3 gebildet, aus der die Einrichtung 1 eine Probe mittels eines Unterdruckgreifers 4 entnimmt. In der Arbeitsstation II wird die Probe auf dem Rand eines weiteren Zylinders 5 mit einer zentralen Öffnung 6 abgelegt, so daß nach Abschalten des Unterdrucks am Greifer ein kleineres Zwischenlagenblatt zur Trennung von der Probe in diese Öffnung fallen kann. Danach wird die Probe mit Hilfe eines mechanischen Greifers 7, der zusammen mit dem Unterdruckgreifer auf eine Wendeplatte 8 der Einrichtung 1 (Roboterkopf) montiert ist, erfaßt und in der Arbeitsstation III auf der Platte einer handelsüblichen elektronischen Papierwaage 9 abgelegt.

Nach der Wägung und Speicherung des Wägeergebnisses wird die Probe mittels des Greifers 7 zur Arbeitsstation IV gebracht, in der sich eine Befeuchtungseinrichtung befindet. Sie besteht aus einem Metallzylinder 10, der in einem durch einen Antrieb 11 in einem Gestell 12 schwenkbaren Rahmen 13 befestigt ist, und einer Platte 14, die mit einem Gummibelag 15 versehen und mittels eines pneumatischen Arbeitszylinders 16 zur Bildung eines bodenseitig abgeschlossenen Behälters 17 dem Metallzylinder 10 zufahrbar ist. Der Arbeitszylinder 16 ist auf einer im Rahmen 13 drehbaren Welle 18 gehalten und durch einen Antrieb 19 um 180° schwenkbar. Nach dem Ablegen der Papierprobe auf dem Gummibelag 15 der Platte 14 mittels des Greifers 7 wird die Platte 14 mit der Probe vom Arbeitszylinder 16 gegen den unteren Rand des Metallzylinders 10 gepreßt, so daß dieser dichtend abgeschlossen ist. Über eine in der Zeichnung nicht dargestellte Brause wird eine definierte Menge Wasser in den Behälter 17 gegossen und dadurch mit der Papierprobe in Kontakt gebracht. Nach einer zu vereinbarenden Einwirkungsdauer wird das Wasser abgegossen, indem der Rahmen 13 mit dem Behälter 17 mit Hilfe des Antriebs 11 soweit geschwenkt wird, daß das Wasser in einen Abflußtrichter 20 fließen kann. Danach schwenkt der Rahmen zurück, worauf die Platte zurückgefahren wird und die befeuchtete Probe vom Greifer 7 der Einrichtung 1 zu einer Ablöscheinrichtung in der Arbeitsstation V gebracht werden kann. Inzwischen dreht der Antrieb 19 den Arbeitszylinder 16 mit der Platte 14 um 180° nach unten, so daß die Platte zum Trocknen des Gummibelags 15 gegen einen Löschkarton 21 gedrückt werden kann. Es ist vorteilhaft, für den Löschkarton eine aus einem Vorrat vorschiebbare Materialbahn vorzusehen.

In der Ablöscheinrichtung 22 wird die Papierprobe 3 auf einer unteren Löschkartonbahn 23 abgelegt, die von einer Vorratsrolle 24 über einen Tisch 25 mit einer Auflagefläche 26 zu einer Aufwickelrolle 27 geführt ist. Über der Auflagefläche ist eine zweite obere Löschkartonbahn 28 parallel zu dieser gespannt, ebenfalls zwischen einer Vorratsrolle 29 und einer Aufwickelrolle 30. Zum Ablöschen der Probe wird die obere Löschkartonbahn mittels einer Ablöschrolle 31 mit der Probe in Kontakt gebracht, die dabei unter ihrem Eigengewicht mehrmals über die abgedeckte Probe rollt. Hierfür ist ein auf zwei Führungsschienen 32 horizontal verschiebbar gelagerter Transportschlitten 33 vorgesehen, der in einer vertikalen Lagernut 34 die Achse 35 der Ablöschrolle 31 lose aufnimmt. Die Löschkartonbahn ist dabei über Umlenkrollen 36 um die Ablöschrolle 31 geführt. Der Antrieb des Transportschlittens erfolgt durch einen in der Zeichnung nicht weiters gezeigten üblichen Reversierantrieb, beispielsweise einen von einem in der Drehrichtung wechselbaren Motor angetriebenen, endlosen Zahnriemen, an dem der Schlitten befestigt ist.

Nach dem Ablöschvorgang befindet sich der Transportschlitten 33 wieder in der Ausgangsstellung, in der die Löschkartonbahn 23 durch eine Senke im Tisch 25 von der Ablöschrolle 31 nicht belastet ist. Die Löschkartonbahn kann dann in Richtung der Vorratsrolle 24 zurückgefahren werden, um die Papierprobe in eine Tasche 37 zu fördern, aus der sie vom Greifer 7 der Einrichtung 1 entnommen und wieder zur Waage 9 gebracht wird. Aus der sich anschließenden Wägung und der anfänglich vorgenommenen ergibt sich dann die Flüssigkeitsaufnahme. Danach wird die Papierprobe an eine Ablagestelle gebracht. Zur Bereitstellung zweier neuer Löschkartonabschnitte für den nächsten Meßzyklus transportieren die Aufwickelrollen 27 und 30 die Bahnen 23 und 28 abschließend in Vorschubrichtung.

Der Antrieb der Vorratsrolle 24 für das Rückwärtsfahren der unteren Bahn 23 und der der Aufwickelrolle 27 erfolgen durch jeweils einen in der Zeichnung nicht zu sehenden Elektromotor, der über im Fachhandel zur Verfügung stehende Kupplungsmittel mit der jeweiligen Rollenwelle verbunden werden kann. Für die obere Löschkartonbahn 28 ist ein gleicher Antrieb nur für die Aufwickelrolle 30 erforderlich.

Die Steuerung für den vorstehend beschriebenen Meßablauf ist nicht Gegenstand vorliegender Erfindung, sondern übliche Steuerungstechnik, so daß auf ihre nähere Erläuterung verzichtet werden kann.

## Ansprüche

1. Meßanordnung zur Bestimmung der Flüssigkeitsaufnahme von Papier, bestehend aus aufeinanderfolgenden Arbeitsstationen (I-V), die eine Waage (9) für eine Papierprobe (3), eine Befeuchtungseinrichtung für die Papierprobe, die durch einen Metallzylinder (10) und eine mit der Papierprobe diesen abschließbaren Platte (14) gebildet ist, und eine Ablöscheinrichtung (22) mit einem oberen und unteren, die Papierprobe einschließenden Löschkarton und eine Ablöschrolle aufweisen, dadurch gekennzeichnet, daß eine von einem Arbeitsprogramm steuerbare, in den Raumkoordinaten (X, Y, Z) den einzelnen Arbeitsstationen zufahrbare Einrichtung (1) mit einer Greifvorrichtung (4, 7) vorgesehen ist, der Waage (9) eine Vorratseinrichtung (2) für die Papierproben (3) vorgeordnet ist, der Metallzylinder (10) und die Platte (14) mittels eines Arbeitszylinders (16) aufeinander zufahrbar sind, wobei der Metallzylinder schwenkbar gelagert ist, und der obere und untere Löschkarton jeweils in Form einer mit einem Materialvorschub verbundenen Bahn (23, 28) über eine Auflagefläche (26) geführt ist, wobei auf der oberen Bahn (28) die Ablöschrolle (31) mittels eines Reversierantriebs abrollbar ist.

2. Meßanordnung nach Anspruch 1, dadurch gekennzeichnet, daß die steuerbare, den einzelnen Arbeitsstationen (I-V) zufahrbare Einrichtung (1) ein Industrieroboter ist.

3. Meßanordnung nach Anspruch 1 und 2, dadurch gekennzeichnet, daß die dem Metallzylinder (10) zugeordnete Platte (14) mittels eines Antriebs (19) um 180° schwenkbar und dadurch mit Hilfe eines Arbeitszylinders (16) wechselweise dem Metallzylinder oder einem Löschkarton (21) zufahrbar ist.

4. Meßanordnung nach Anspruch 1 bis 3, dadurch gekennzeichnet, daß die obere und die untere Löschkartonbahn (28, 23) im Abstand zueinander und parallel zur Auflagefläche (26) geführt sind und die obere Bahn (28) dabei um die in einem Transportschlitten (33) vertikal lose gelagerte, auf der Auflagefläche abgestützte Ablöschrolle (31) geführt ist.

I

2

3

II

6

5

III

9

7 8

1

4

237/88

V

29 28 22

36 34 31

25 30

37 3 23

24 27

32 33 35

26

IV

17

10

11

15 13

14

18 12

20 19

16 21

EP 0 344 554 A2